(19) Europäisches Patentamt / European Patent Office / Office européen des brevets

(11) EP 1 682 118 B1

(12) **EUROPEAN PATENT SPECIFICATION**

(45) Date of publication and mention of the grant of the patent:
**22.10.2008 Bulletin 2008/43**

(21) Application number: **04817400.7**

(22) Date of filing: **03.11.2004**

(51) Int Cl.:
***A61K 31/365*** *(2006.01)* ***A61K 31/366*** *(2006.01)*
***A61K 31/415*** *(2006.01)* ***A61K 31/4164*** *(2006.01)*
***A61K 31/4192*** *(2006.01)* ***A61P 33/00*** *(2006.01)*
***A61P 33/14*** *(2006.01)*

(86) International application number:
**PCT/EP2004/052762**

(87) International publication number:
**WO 2005/041950 (12.05.2005 Gazette 2005/19)**

(54) **ECTOPARASITICIDAL FORMULATIONS OF SPINOSYNS AND AZOLE PESTICIDES**

ECTOPARASITISCHE FORMULIERUNGEN VON SPINOSYNS UND AZOL-PESTIZIDEN

PREPARATIONS ECTOPARASITICIDES DE SPINOSYNES ET DE PESTICIDES A BASE D'AZOLE

(84) Designated Contracting States:
**AT BE BG CH CY CZ DE DK EE ES FI FR GB GR HU IE IS IT LI LU MC NL PL PT RO SE SI SK TR**

(30) Priority: **04.11.2003 EP 03078569**

(43) Date of publication of application:
**26.07.2006 Bulletin 2006/30**

(73) Proprietor: **Intervet International BV**
**5831 AN Boxmeer (NL)**

(72) Inventors:
- **MERTENS, Christina**
  **55270 Engelstadt (DE)**
- **DOHRMANN, Heike**
  **55270 Sörgenloch (DE)**
- **RSHAID, Gabriel Aldolfo Marcos**
  **NL-6866 CG Heelsum (NL)**

(74) Representative: **Stumm, Karin et al**
**Intervet International B.V.**
**P.O. Box 31**
**5830 AA Boxmeer (NL)**

(56) References cited:
**EP-A- 0 412 849**
**WO-A-01/11963**
**WO-A-94/20518**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 682 118 B1

## Description

The present invention relates to formulations comprising a combination of different active ingredients with antiparasitic activity for the control of ectoparasites, and to a method for the manufacture of a medicament for controlling an ectoparasite infestation by administering the active ingredients in combination, either simultaneously or sequentially.

A number of pests and parasites can infest or infect domestic animals such as cattle, horses, pigs, sheep, but also companion animals such as cats and dogs. These pests and parasites are of great nuisance to both the animals and their owners. External parasites such as ticks, mites, lice and fleas irritate the animals and can cause disease, either by themselves, or by carrying vector transmitted pathogens.

Ticks are important blood feeding arthropod parasites that belong together with mites to the order *Acarina.* There are two well-established families of ticks, the *Ixodidae* (hard ticks), and *Argasidae* (soft ticks).
Ticks produce injury after infestation of animals in three respects: direct damage caused by parasitism such as local injury and blood loss; by toxins injected by the parasites and by the transmission of diseases. Especially in companion animals, ticks may be the source of zoonotic diseases.

Many mites, of the order of prostigmata and astigmata, as a result of their biting or feeding habits, can cause serious illness and are a serious nuisance to their hosts.

Fleas are the most common ectoparasites of cats and dogs, *Ctenocephalides felis felis* and *Ctenocephalides canis,* respectively. However, cat fleas also infest dogs. The cat flea can also transmit disease such as tapeworm in dogs.

Safe, effective ways to eliminate these parasites are desired both for the companion animal's well being and for well-being and the comfort of its human associate and for the prevention of losses in livestock.

Thus, there continues to be a need for compounds and combinations thereof which can be used as active agents against ectoparasites, especially those that afflict domestic animals, and which are effective at low application rates, selective in biologic action and have low toxicity.

The present invention now provides antiparasitic combinations of spinosyns with azole pesticides for the control of ectoparasites.

The spinosyns (also known as A83453 factors) are agricultural insecticides that have shown activity against southern armyworm and other insects in the order *Lepidophtera,* and cotton aphid and other members of the order *Homopthera* (see for example U.S. Patent No. 5,571,901). The spinosyns were also known to have some ectoparasiticidal activity, i.e. *in vitro* activity against mosquito larvae, black blowfly larvae and adult stable flies, which are members of the insect order *Diptera,* and transient systemic activity against larval blowfly and adult stable fly in guinea pigs and sheep (see U.S. Patent No. 5,571,901).

The fermentation product A83543, also known as spinosyn, includes a family of related compounds (spinosyns) produced by *Saccharopolyspora spinosa.* These are naturally derived fermentation products with a positive safety profile and have previously been shown to exhibit good insecticidial activity. Spinosyns are also known as "A83543 compounds" and are compounds consisting of a 5,6,5-tricyclic ring system, fused to a 12-membered macrocyclic lactone, a natural sugar (2N,3N,4N-tri-O-methylrhamnose) and an amino sugar (fructosamine). The various spinosyns are characterized by differences in the substitution patterns on the amino group of the forosamine, at selected sites on the tetracyclic ring system and on the 2N, 3N, 4N - (tri-O-methyl) rhamnose group.

The use of spinosyns in oral preparations for the treatment of dogs against ectoparasites (ticks) is mentioned in WO01/11963. The spinosyns were used at relatively high dosage, but showed only limited activity against the ticks.

Azole pesticides are disclosed in EP 412849. EP412849 discloses certain aryl-1,2,3 triazoles and arylpyrazoles in which the imidazol(in)e group is attached directly or indirectly through its 2-position to the triazole or pyrazole ring that have pesticidal activity.

It has now been found that by combination of an azole pesticide of the type described in EP412849, and a pesticide of the spinosyn type, a broad spectrum ectoparasitic formulation can be obtained that is highly effective at low dose.

The present invention therefore provides an antiparasitic formulation characterized in that it comprises a combination of one or more spinosyns with a compound of formula (1)

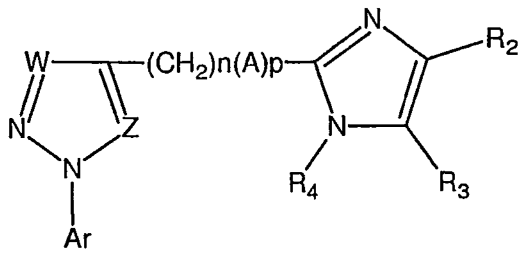

(I)

and salts thereof, in which

Ar is 2,6-dichloro-4- trifluoromethylphenyl or 2-nitro-4-trifluoromethylphenyl;
A is $S(O)_m$, -CH=CH-, O or NH;
W is N and Z is $CR^5$; or W is $CR^1$ and Z is N or $CR^5$;
$R^1$ is hydrogen, optionally substituted alkyl, halogen or $R^{20}S(O)_q$;
$R^2$ and $R^3$ are hydrogen, alkyl, alkenyl or alkynyl, each of which is optionally substituted, aryl, cyano, halogen, nitro, $YR^{20}$, $S(O)_2NR^8R^9$, CHO, $NR^8R^9$ or $CYNR^8R^9$; $R^4$ is hydrogen, optionally substituted alkyl, optionally substituted alkenyl, acyl or optionally substituted alkoxycarbonyl;
$R^5$ is hydrogen, alkyl, optionally substituted amino or halogen;
$R^8$ and $R^9$ are the same or different and are hydrogen, optionally substituted alkyl, acyl or aryl;
$R^{20}$ is optionally substituted alkyl;
Y is O orS;
m is 0, 1 or 2;
p is 0 or 1;
n is 0, 1 or 2; and
q is 0, 1 or 2,
and in which a) any alkyl, alkoxy and alkylthio groups is of 1 to 4 carbon atoms; b) any alkenyl or alkynyl groups is of 2 to 5 carbon atoms; c) any substituted alkyl, alkoxy, alkylthio, alkenyl or alkynyl group is substituted by one or more of the same or different groups selected from halogen, $YR^{20}$, dihalocyclopropyl, cyano, nitro, optionally substituted amino, acyloxy and aryl; d) any aryl group is phenyl, optionally substituted, by halogen, alkyl, haloalkyl, alkoxy, haloalkoxy, alkylthio, haloalkylthio, haloalkylsulphonyl, cyano or nitro; e) any acyl group is alkanoyl of 1 to 4 carbon atoms, or alkylsulphonyl or haloalkylsulphonyl; and f) any optionally substituted amino groups is of formula $NR^8R^9$, with the proviso that when W is $CR^1$ and Z is $CR^5$ and n and p are both 0, $R^4$ is not alkyl.
$R^4$ is preferably hydrogen. When W is $CR^1$ and Z is $CR^5$ and n and p are both 0, but also for all other compounds, it is preferred that at least one, and especially both, of
$R^2$ and $R^3$ are cyano. n and p are preferably 0. $R^5$ is preferably halogen, especially chlorine.

The azole pesticides described above have been described in EP412849. Methods of how these compounds can be prepared are disclosed in EP412849 as well.

An especially preferred compound for use in the formulations according to the invention is 5-chloro-1-(2,6,dichloro-4-trifluoromethylphenyl)-4-(4,5-dicyano-1H-imidazol-2-yl)-3-isopropyl-1H-pyrazole, which is the compound indicated as compound 22c in EP412849. The synthesis of this compound is disclosed in example 22b of EP412849. In the remainder of this application this compound will be referred to as "compound 22c".

As far as the spinosyns used in the formulations according to the invention are concerned, particular preference is given to using Spinosad (see for example DowElanco trade magazine Down to earth Vol. 52, No. 1, 1997 and literature cited therein), which essentially consists of a mixture of spinosyn A and spinosyn D in a ratio of approximately 85:15. Use is made, in particular; of the fermentation product A 83543 known from U.S. Patent No. 5,362,634 which comprises approximately 85 to 90% of spinosyn A, approximately 10 to 15% of spinosyn D and smaller amounts of the spinosyns B, C, E, F, G, H and J.

The spinosyns can react to form salts that are also useful in the present invention. The salts are prepared using standard procedures for salt preparation. For example, spinosyn A can be neutralized with an appropriate acid to form an acid addition salt. The acid addition salts of spinosyns are particularly useful. Representative suitable acid addition salts include salts formed by reaction with either an organic or inorganic acid.

A most preferred combination is a combination of compound 22c and spinosad.

The active ingredients do not necessarily need to be part of one and the same physical preparation. They may also be part of separate preparations.

The active ingredients may be administered at the same time, that is simultaneously, but they may also be administered sequentially, that is one after the other. If given sequentially, one active ingredient is detectable on/in the host animal when the other is applied.
Thus, in another embodiment the invention relates to the use of one or more spinosyns and at least one compound according to formula 1 as active ingredients in a method for the manufacture of a medicament for controlling an ectoparasite infestation by administering the active ingredients in combination, either simultaneously or sequentially.
The active ingredients are preferably administered simultaneously. When given simultaneously the combination is preferably presented as a formulation, which is a single physical preparation comprising both the compound of formula 1 and the spinosyn(s).
The formulations of the invention are intended for use for controlling an ectoparasitic infestation. The term "controlling an ectoparasite infestation" refers to preventing, minimizing or eliminating an infestation by an ectoparasite. The term "ectoparasite" refers to insect and acarine pests that commonly infest or infect animals. Examples of such ectoparasites include the egg, larval, pupal, nymphal and adult stages of lice, flea, mosquitoes, mites, ticks and blood-sucking, biting or nuisance fly species.
In general, the formulations according to the invention will contain an effective amount of the active ingredients, meaning a non-toxic but sufficient amount to provide the desired therapeutic effect. A person skilled in the art using routine experimentation may determine an appropriate "effective" amount in any individual case. Such an amount will depend on the age, condition, weight and type of the target animal.
The animals may receive a monthly, weekly or daily dosage, optionally preceded by a higher loading dose (initial higher dose). The treatment can, for example, be continuing or seasonal.
The ratio between the compound of formula (I) and one or more spinosyns when used in combination is preferably 1:10 to 10:1.
Preferably the treatment is carried out so as to administer to the animal a dose from 0.1 to 100 and in particular from 1 to 40 mg /kg bodyweight of the compound of formula (I) and a dose from 0.1 to 100 and in particular 1 to 40 and most preferably less than 30 mg/ kg bodyweight of the spinosyn compound(s).
These dosages have been proven to be effective, especially in companion animals such as dogs.
Preferably the combination of active ingredients is administered systemically. Systemic administration is the administration of the combination at a site remote from the site where the parasite resides, e.g. to an animal by mouth, by injection, by implants, or by other means, for example transdermal delivery such as spot-on or pour-on, so that there is sufficient of the agent in the tissues or body fluids to kill the parasite feeding on the animal.
The formulations of the invention are preferably administered in an oral formulation. The term "oral formulation" means that the active ingredients are formulated into a product suitable for administering to the animal via the mouth. These formulations include, but are not limited to tablets, capsules, liquids, gels, pastes, oral sprays, buccal formulations, powders, granules, chewable treats or animal feeds containing the active ingredients. Generally such formulations include a physiologically acceptable carrier. Such carriers are well known in the veterinary art.
Conventional tablets generally comprises the active ingredients, a diluent to assist in increasing the powder mass to a convenient size and improve compressability, a binder to hold the compressed powder together and a lubricant to assist in densification and ejection from the tablet die. They may also contain a disintegrate, to improve disintegration and dissolution as well as stabilizers, colours and flavours. Tablets are often coated to improve appearance or taste or to alter the dissolution properties. Tablets can be designed to dissolve fast or slow, and depending on the actual volume and compressability of the drug, large or small. They can be made chewable or to dissolve under the tongue or in the pouch of the cheek. Conventional liquid formulations are usually solutions, suspension or emulsions of the component together with suitable diluents, solvents, flavours and colours to form a palatable dosage form. Preferably the formulations according to the invention are provided as tablets, preferably chewable tablets that can be given to, for example dogs, as a treat. The tablets may be formulated to contain an amount of active ingredients that is adjusted to animals in a specific weight range.
In general the formulation according to the current invention can be administered to all species of animals that have ectoparasite infestation. The recipient of the formulation may be livestock animal e.g. sheep, cattle, pig, goat, poultry, a laboratory test animal, e.g. guinea pig, rat or mouse or a companion animal e.g. dog, cat rabbit or horse. The formulations according to the invention are especially suitable for use in companion animals, e.g dogs.

EXAMPLES:

<u>Example 1: Efficacy and tolerability of Compound 22c in combination with Spinosad.</u>

Two groups of 6 dogs each (group A and B) were infested with fleas (*Ctenocephalides felis*) and ticks (*Rhipicephalus sanguineus*) before and repeatedly, at different time points after treatment.
Dogs in group B served as an untreated control, dogs in group A were orally treated with Compound 22c in combination

with spinosad. For Compound 22c an initial dose of 4 mg/kg bodyweight (bw) was used on day 0 (d0), followed by 3 weekly doses (d+7, d+14, d+21) of 2 mg/kg bw. Spinosad was given at an initial dose of 30 mg/kg at d0, followed by weekly doses of 15mg/kg on d+7, d+14 and d+21.

The parasite burden of individual dogs was assessed 48 hours after each infestation by removing and counting of ticks and fleas. Fleas and ticks were classed according to vitality (dead / alive) and the parasitic status of ticks (engorged / non-engorged; attached / free). Parasite numbers were used to calculate efficacy. The combination of Compound 22c and spinosad was well tolerated throughout the study.

The details of the Study procedures are indicated below:

STUDY PROCEDURES (Materials and Methods)

Product specifications

<u>Compound 22c Tablet</u>

| | |
|---|---|
| Active Ingredient: | Compound 22c (Ch.HT/a 99.1%) |
| Product formula: | Compound 22c 10.0 % (w/w) |
| Dosage: | Orally: 4 mg/kg body mass initial dose; 2 mg/kg body mass maintenance dose |
| Application: | Per os |

<u>Spinosad capsule</u>

| | |
|---|---|
| Active ingredient: | 99% |
| Formulation: | 87% Spinosyn A<br>12% Spinosyn D |
| Dosage: | Orally: 30mg/kg body mass initial dose; 15 mg/kg body mass maintenance dose |
| Application: | Per os (in gelatine capsules) |

Study animals

| | |
|---|---|
| Species: | Domestic dog |
| Number: | 12 |
| Health status: | The dogs were healthy at the start of the study as determined by the veterinarian on day -2. None of the dogs had been treated with an acaricide/insecticie for at least 8 weeks prior to the study. |
| Identification: | Ear and chip number |

Animal Housing

Study animals were kept indoors in individual pens with concrete floors and a special resting area allowing the collection of parasites from the environment. The study was carried out in accordance with the existing guidelines for the "Testing and evaluation of the efficacy of antiparasitic substances for the treatment and prevention of tick and flea infestation in dogs and cats" (EMEA/CVMP/005/00) providing maximum comfort to the dogs under study conditions.

Parasite infestation

Dogs were experimentally infested with laboratory strains of *Rhipicephalus sanguineus* (80 unfed adults; sex

ratio 1:1) (ticks) and *Ctenocephalides felis* (100 adults, similar age; sex ratio approx. 1:1) (fleas) according to table 1. Fleas and ticks were directly applied onto the back of the animal. Ticks (*Rhipicephalus sanguineus*) and fleas (*Ctenocephalides felis*) were infested on day 2, day +3, day +5, day +7, day +10, day +12, day +14, day +17, day +19, day +21, day + 24, day + 26.

Treatment

Dogs in group A were treated according to the schedule as set out in Table 1. Dogs in Group B were infested but not treated and served as untreated control.

Route and method of Administration

To calculate the doses for Compound 22c the body weights were used. The smallest unit that had been used was half a tablet Compound 22c. Tablets were not scored but were broken into even pieces by using a tablet-cutter. Where necessary the dose was rounded to the nearest half tablet. Spinosad was given as a single capsule. Before each treatment, the individual dose for each dog was weight into the capsule according to the actual body weight.

Both products were given to the dogs hidden in a small amount of dog-food. It was tested before the application if the individual dog would accept the food. Both products were applied directly to the back of the tongue to induce swallowing when the dog would refuse the food. The administered doses were recorded.

TABLE 1: Infestation, treatment and assessment scheme.

| Day | Infestation | | Assessment | | Treatment | |
|---|---|---|---|---|---|---|
| | *Ticks* | *Fleas* | *Ticks* | *Fleas* | *Compound 22c* | *Spinosad* |
| *d-2* | 80 | 100 | | | | |
| *d-1* | | | | | | |
| *d0* | | | | | 4 mg/kg | 30 mg/kg |
| *d1* | | | | | | |
| *d2* | | | X | X | | |
| *d3* | 80 | 100 | | | | |
| *d4* | | | | | | |
| *d5* | 80 | 100 | X | X | | |
| *d6* | | | | | | |
| *d7* | 80 | 100 | X | X | 2 mg/kg | 15mg/kg |
| *d8* | | | | | | |
| *d9* | | | X | X | | |
| *d10* | 80 | 100 | | | | |
| *d11* | | | | | | |
| *d12* | 80 | 100 | X | X | | |
| *d13* | | | | | | |
| *d14* | 80 | 100 | X | X | 2 mg/kg | 15 mg/kg |
| *d15* | | | | | | |
| *d16* | | | X | X | | |
| *d17* | 80 | 100 | | | | |
| *d18* | | | | | | |
| *d19* | 80 | 100 | X | X | | |
| *d20* | | | | | | |

(continued)

| Day | Infestation | | Assessment | | Treatment | |
|---|---|---|---|---|---|---|
| | *Ticks* | *Fleas* | *Ticks* | *Fleas* | *Compound 22c* | *Spinosad* |
| *d21* | 80 | 100 | X | X | 2 mg/kg | 15 mg/kg |
| *d22* | | | | | | |
| *d23* | | | X | X | | |
| *d24* | 80 | 100 | | | | |
| *d25* | | | | | | |
| *d26* | 80 | 100 | X | X | | |
| *d27* | | | | | | |
| *d28* | | | X | X | | |
| *d30* | | | | | | |
| *d33* | | | | | | |

Evaluation of Tick and Flea Numbers

**[0043]** Parasite assessments on the dogs were made 48 hours after the first treatment and after each re-infestation, as indicated in table 1.
The flea count on each dog was conducted by combing until no flea was recovered for at least 5 minutes and the number of live fleas was recorded. Ticks on the dog were counted by palpation, removed where attached and classified according to the categories indicated in Table 2

TABLE 2: Tick categories

| Category | General findings | Attachment status | Effect |
|---|---|---|---|
| 1 | Live | Free | No |
| 2 | Live | Attached - unengorged | No (except single ticks) |
| 3 | Live | Attached — engorged | No (except single ticks) |
| 4 | Killed | Free | Yes |
| 5 | Killed | Attached — unengorged | Yes |
| 6 | Killed | Attached — engorged | No (except single ticks) |

Evaluation of Clinical Symptoms

During the daily routine behaviour and condition.

Calculation of Efficacy

Efficacy calculation was based on the arithmetic means of number of ticks / fleas on treated dogs compared to the control group. For calculation of efficacy (%), the following formula (according to Abott's formula) is used:

$$\text{Efficacy} = 100 \times (mc - mt)/ mc$$

Control group (mc): mean number of live fleas on the host animal
mean number of live ticks on the host animal

Treatment group (mt): mean number of live fleas on the host animal

mean number of live (category 1-3) or killed, engorged ticks (category 6) on the host animal

RESULTS:

The calculated efficacies are reflected in the table below (Table 3).

TABLE 3: Efficacy of treatment with combination of Compound 22c and Spinosad.

| *Day* | *GROUP A* | | *GROUP B (control)* | |
|---|---|---|---|---|
| | *Ticks* | *Fleas* | *Ticks* | *Fleas* |
| d+2 | 99.3 | 100 | 0 | 0 |
| d+5 | 99.3 | 100 | 0 | 0 |
| d+7 | 95.7 | 100 | 0 | 0 |
| d+9 | 100 | 100 | 0 | 0 |
| d+12 | 99.6 | 100 | 0 | 0 |
| d+14 | 99.6 | 100 | 0 | 0 |
| d+16 | 99.6 | 100 | 0 | 0 |
| d+19 | 99.7 | 100 | 0 | 0 |
| d+21 | 100 | 100 | 0 | 0 |
| d+23 | 100 | 100 | 0 | 0 |
| d+26 | 100 | 100 | 0 | 0 |
| d+28 | 99.4 | 100 | 0 | 0 |

From these results it can be concluded that the combined treatment with Compound 22c and Spinosad resulted in an outstanding efficacy against ticks as well as fleas.

Example 2: Dose titration of (combination of) compound 22c and Spinosad

An in vitro model was used to evaluate compound 22c, spinosad, and a combination thereof for acaricidal activity at several concentrations when being ingested by the parasite. The test system is the unfed nymph (3rd stage) of *Ornithodorus moubata* (soft tick).

*Preparation of test solutions:*

Test compounds were dissolved in DMSO/Emulsogen (standard preparation) and defibrinised sheep blood as follows:

20 µl DMSO + 980 µl blood: 1 mg compound 22c
1 mg Spinosad
1 mg compound 22c plus 1 mg Spinosad (1+1)
1 mg compound 22c plus 2 mg Spinosad (1 +2)
40 µl DMSO + 960 µl blood: 1 mg compound 22c plus 4 mg Spinosad (1+ 4)
1 mg compound 22c plus 6 mg Spinosad (1+6).

The defibrinised sheep blood was added to the DMSO-compound preparations and thoroughly mixed. The appropriate test concentrations were prepared by dilutions of this starting solution of 1000 ppm in defibrinised sheep blood. The dilutions are thoroughly mixed and prepared freshly before the feeding procedure.

The specific activity is determined at a dilution range of concentrations.

*Feeding procedure:*

For each concentration, at least 20 nymphs of O. moubata (stage III) were fed via a parafilm membrane on 2 ml defibrinised sheep blood supplemented with the test compound at the appropriate concentrations and heated to 38 — 40°C. Feeding was completed after approximately 30 minutes. The test organisms were then transferred to Petri dishes and incubated at 27 — 29°C and about 80 % relative humidity for up to four weeks.
The following parameters were assessed:

feeding (acceptance of the diet)
phenotype (morphological damage, behaviour)
developmental defects (molting)
mortality

*Assessment:*

Activity was assessed by mortality (%) and inhibition of development into the stage IV nymph: Feeding was monitored during the tick feeding procedure. Phenotype and mortality of 20 engorged nymphs were assessed during and after feeding, 24 hours after feeding and approximately 28 days after feeding. The mortality is expressed in percent in relation to a non-medicated control. The percentages are reflected in table 4.
From Table 4 it can be seen that both compounds attribute to the antiparasitic effect of the mixture. The mixtures are still active at lower concentrations for compound 22 C in the mixture, than compound 22c (and spinosad) alone.

Table 4: Dose-titration for 22c, spinosad, and mixtures thereof.

| | Dilution range of starting solution : | 1:1 (starting solution) | 1:2 | 1:5 | 1:10 | 1:20 | 1:50 | 1:100 | 1:500 | 1:1000 | 1:5000 |
|---|---|---|---|---|---|---|---|---|---|---|---|
| | Compound(s) (starting solutions) | | | | | | | | | | |
| 1 | 22c: 0 ppm<br>SP: 50 ppm | 100 | 95 | 85 | 45 | 30 | 10 | 5 | 5 | 5 | 5 |
| 2 | 22c: 50ppm<br>SP: 0 ppm | 100 | 100 | 100 | 100 | 20 | 10 | 10 | 5 | 5 | 0 |
| 3 | 22c: 50 ppm<br>SP: 50 ppm | 100 | 100 | 100 | 100 | 100 | 100 | 50 | 15 | 0 | 0 |
| 4 | 22c : 50 ppm<br>SP: 100 ppm | 100 | 100 | 100 | 100 | 100 | 100 | 55 | 10 | 0 | 0 |
| 5 | 22c 50 ppm<br>SP: 200 ppm | 100 | 100 | 100 | 100 | 100 | 100 | 75 | 50 | 15 | 0 |
| 6 | 22c : 50 ppm<br>SP: 300 ppm | 100 | 100 | 100 | 100 | 100 | 100 | 90 | 35 | 30 | 10 |
| | | | | | | | | | | | |
| 7 | Control (blood only) | 0 | 10 | 0 | 5 | 0 | 0 | 0 | 5 | 0 | 5 |
| 8 | Solvent control: blood plus DMSO/Emulsogen | 5 | 0 | 0 | 0 | 5 | 10 | 5 | 0 | 0 | 0 |

Example 3: Efficacy of Spinosad alone in the control of ticks (Comparative example)

In an experiment that was carried out in essentially the same setting as the experiment described in Example 1. Group A consisted of two dogs, treated with with Spinosad alone against infestation with ticks (*Rhipicephalus sanguineus*). In group A, one dog was treated with 50mg Spinosad per kg body weight (bw), and the other dog was treated with 15 mg Spinosad per kg bw.
The two dogs in group B served as untreated control.
The time plan for the experiment is indicated in Table 5, and the results are indicated in Table 6:

Table 5: Infestation, treatment and assessment scheme (spinosad only)

| Day | Infestation | Assessment | treatment |
|---|---|---|---|
| | ticks | | Spinosad |
| d-2 | 80 | | |
| d-1 | | | |
| D0 | | | 50 or 15 mg/kg |
| D1 | | | |
| D2 | | X | |
| D3 | 80 | | |
| D4 | | | |
| D5 | 80 | X | |
| D6 | | | |
| D7 | | X | |

RESULTS:

TABLE 6: Efficacy of treatment against ticks with Spinosad.

| Day | GROUP A | | GROUP B (CONTROL) |
|---|---|---|---|
| | 50mg/kg | 15mg/kg | |
| d+2 | 96.9 | 60.9 | 0 |
| d+5 | 62.5 | 0 | 0 |
| d+7 | 62.6 | 0 | 0 |

From the results it shows that 1 week treatment with 15 mg/kg spinosad was insufficient to control ticks, whereas treatment with 50 mg/kg spinosad was not sufficient to prevent the reinfestation with ticks.

## Claims

1. An antiparasitic formulation **characterized in that** it comprises, as active ingredients, spinosyn(s) in combination with a compound of formula (I)

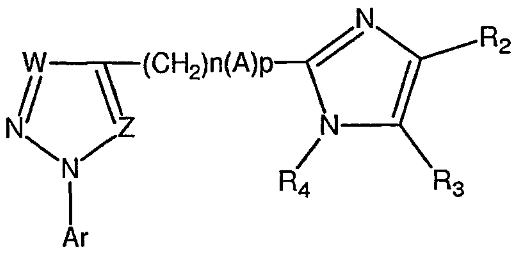

(I)

and salts thereof, in which

Ar is 2,6-dichloro-4- trifluoromethylphenyl or 2-nitro-4-trifluoromethylphenyl;
A is $S(O)_m$, -CH=CH-, O or NH;
W is N and Z is $CR^5$; or W is $CR^1$ and Z is N or $CR^5$;
$R^1$ is hydrogen, optionally substituted alkyl, halogen or $R^{20}S(O)_q$;
$R^2$ and $R^3$ are hydrogen, alkyl, alkenyl or alkynyl, each of which is optionally substituted, aryl, cyano, halogen, nitro, $YR^{20}$, $S(O)_2NR^8R^9$, CHO, $NR^8R^9$ or $CYNR^8R^9$; $R^4$ is hydrogen, optionally substituted alkyl, optionally substituted alkenyl, acyl or optionally substituted alkoxycarbonyl;
$R^5$ is hydrogen, alkyl, optionally substituted amino or halogen;
$R^8$ and $R^9$ are the same or different and are hydrogen, optionally substituted alkyl, acyl or aryl;
$R^{20}$ is optionally substituted alkyl;
Y is O or S;
m is 0, 1 or 2;
p is 0 or 1;
n is 0, 1 or 2; and
q is 0, 1 or 2,

and in which a) any alkyl, alkoxy and alkylthio groups is of 1 to 4 carbon atoms; b) any alkenyl or alkynyl groups is of 2 to 5 carbon atoms; c) any substituted alkyl, alkoxy, alkylthio, alkenyl or alkynyl group is substituted by one or more of the same or different groups selected from halogen, $YR^{20}$, dihalocyclopropyl, cyano, nitro, optionally substituted amino, acyloxy and aryl; d) any aryl group is phenyl, optionally substituted, by halogen, alkyl, haloalkyl, alkoxy, haloalkoxy, alkylthio, haloalkylthio, haloalkylsulphonyl, cyano or nitro; e) any acyl group is alkanoyl of 1 to 4 carbon atoms, or alkylsulphonyl or haloalkylsulphonyl; and f) any optionally substituted amino groups is of formula $NR^8R^9$, with the proviso that when W is $CR^1$ and Z is $CR^5$ and n and p are both 0, $R^4$ is not alkyl.

**2.** The formulation according to claim 1 **characterized in that** the compound of formula (I) is 5-chloro-1-(2,6-dichloro-4-trifluoromethylphenyl)-4-(4,5-dicyano-1 H-imidazol-2-yl-3-methyl-1-H pyrazole (compound 22c).

**3.** The formulation according to claim 1 or 2 **characterized in that** the spinosyns are a mixture of any two or more spinosyn compounds.

**4.** The formulation according to claim 3 **characterized in that** the mixture is spinosad.

**5.** The formulation according to any of claims 1-4 **characterized in that** the compound of formula (I) and one or more spinosyns are present in a ratio of 1:10 to 10:1.

**6.** Use of one or more spinosyns and at least one compound according to formula 1 as active ingredients in the manufacture of a medicament for controlling an ectoparasite infestation by administering the active ingredients in combination, either simultaneously or sequentially.

**7.** Use according to claim 6 wherein the active ingredients are 5-chloro-1-(2,6-dichloro-4-trifluoromethylphenyl)-4-(4,5-dicyano-1H-imidazol-2-yl-3-methyl-1-H pyrazole and spinosad.

**8.** Use according to claim 6 or 7, wherein the medicament is for controlling an ectoparasite infestation by administering the active ingredients simultaneously.

**9.** Use according to claim 8, wherein the medicament comprises the active ingredients in a single preparation.

**10.** Use according to claim 6 wherein the ectoparasites are ticks.

**11.** Use according to claim 6 wherein the ectoparasites are fleas.

**Patentansprüche**

**1.** Antiparasitische Formulierung, **dadurch gekennzeichnet, daß** sie als Wirkstoffe Spinosyn(e) in Kombination mit einer Verbindung der Formel (I)

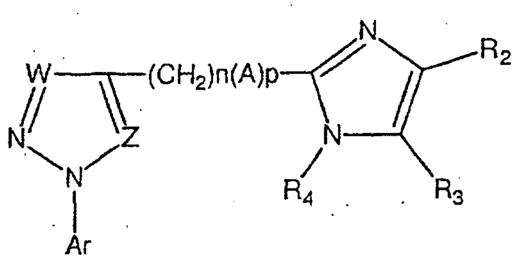

(I)

und ihren Salzen umfaßt, wobei

Ar 2,6-Dichlor-4-trifluormethylphenyl oder 2-Nitro-4-trifluormethylphenyl bedeutet;
A $S(O)_m$, -CH=CH-, O oder NH bedeutet;
W N bedeutet und Z $CR^5$ bedeutet; oder W $CR^1$ bedeutet und Z N oder $CR^5$ bedeutet;
$R^1$ Wasserstoff, gegebenenfalls substituiertes Alkyl, Halogen oder $R^{20}S(O)_q$ bedeutet;
$R^2$ und $R^3$ Wasserstoff, Alkyl, Alkenyl oder Alkinyl, das jeweils gegebenenfalls substituiert ist, Aryl, Cyano, Halogen, Nitro, $YR^{20}$, $S(O)_2NR^6R^9$, CHO, $NR^8R^9$ oder $CYNR^8R^9$ bedeutet;
$R^4$ Wasserstoff, gegebenenfalls substituiertes Alkyl, gegebenenfalls substituiertes Alkenyl, Acyl oder gegebenenfalls substituiertes Alkoxycarbonyl bedeutet;
$R^5$ Wasserstoff, Alkyl, gegebenenfalls substituiertes Amino der Halogen bedeutet;
$R^8$ und $R^9$ gleich oder verschieden sind und Wasserstoff, gegebenenfalls substituiertes Alkyl, Acyl oder Aryl bedeuten;
$R^{20}$ gegebenenfalls substituiertes Alkyl bedeutet;
Y O oder S bedeutet;
m 0, 1 oder 2 bedeutet;
p 0 oder 1 bedeutet;
n 0, 1 oder 2 bedeutet; und
q 0, 1 oder 2 bedeutet,

und wobei a) jede Alkyl-, Alkoxy und Alkylthiogruppe 1 bis 4 Kohlenstoffatome umfaßt; b) jede Alkenyl- oder Alkinylgruppe 2 bis 5 Kohlenstoffatome umfaßt; c) jede substituierte Alkyl-, Alkoxy-, Alkylthio-, Alkenyl- oder Alkinylgruppe durch eine oder mehrere gleiche oder verschiedene Gruppen aus der Reihe Halogen, $YR^{20}$ Dihalogencyclopropyl, Cyano, Nitro, gegebenenfalls substituiertes Amino, Acyloxy und Aryl substituiert ist; d) jede Arylgruppe Phenyl, das gegebenenfalls durch Halogen, Alkyl, Halogenalkyl, Alkoxy, Halogenalkoxy, Alkylthio, Halogenalkylthio, Halogenalkylsulfonyl, Cyano oder Nitro substituiert ist; e) jede Acylgruppe Alkanoyl mit 1 bis 4 Kohlenstoffatomen, oder Alkylsulfonyl oder Halogenalkylsulfonyl bedeutet; und f) jede gegebenenfalls substituierte Aminogruppe der Formel $NR^8R^9$ entspricht, mit der Maßgabe, daß, wenn W $CR^1$ bedeutet und Z $CR^5$ bedeutet und n und p beide 0 bedeuten, dann $R^4$ nicht Alkyl bedeutet.

**2.** Formulierung nach Anspruch 1, **dadurch gekennzeichnet, daß** es sich bei der Verbindung der Formel (I) um 5-Chlor-1-(2,6-dichlor-4-trifluormethylphenyl)4-(4,5-dicyano-1H-imidazol-2-yl-3-methyl-1H-pyrazol (Verbindung 22c) handelt.

**3.** Formulierung nach Anspruch 1 oder 2, **dadurch gekennzeichnet, daß** es sich bei den Spinosynen um eine Mischung von beliebigen zwei oder mehr Spinosynverbindungen handelt.

**4.** Formulierung nach Anspruch 3, **dadurch gekennzeichnet, daß** es sich bei der Mischung um Spinosad handelt.

**5.** Formulierung nach einem der Ansprüche 1-4, **dadurch gekennzeichnet, daß** die Verbindung der Formel (I) und eine oder die mehreren Spinosyne im Verhältnis 1:10 bis 10:1 vorliegen.

**6.** Verwendung von einem oder mehreren Spinosynen und mindestens eine Verbindung der Formel (I) als Wirkstoffe bei der Herstellung eines Arzneimittels für die Bekämpfung eines Befalls mit Ektoparasiten **dadurch**, daß man die Wirkstoffe in Kombination, und zwar entweder gleichzeitig oder nacheinander, verabreicht.

**7.** Verwendung nach Anspruch 6, wobei es sich bei den Wirkstoffen um 5-Chlor-1-(2,6-dichlor-4-trifluormethylphenyl)-4-(4,5-dicyano-1H-imidazol-2-yl-3-methyl-1-H-pyrazol und Spinosad handelt.

**8.** Verwendung nach Anspruch 6 oder 7, wobei das Arzneimittel der Bekämpfung von Befall mit Ektoparasiten durch gleichzeitige Verabreichung der Wirkstoffe dient.

**9.** Verwendung nach Anspruch 8, wobei das Arzneimittel die Wirkstoffe in einem einzigen Präparat umfaßt.

**10.** Verwendung nach Anspruch 6, wobei es sich bei den Ektoparasiten um Zecken handelt.

**11.** Verwendung nach Anspruch 6, wobei es sich bei den Ektoparasiten um Flöhe handelt.

**Revendications**

**1.** Formulation antiparasitaire, **caractérisée en ce qu'**elle comprend, à titre de principes actifs, une ou des spinosynes en combinaison avec un composé de formule (I)

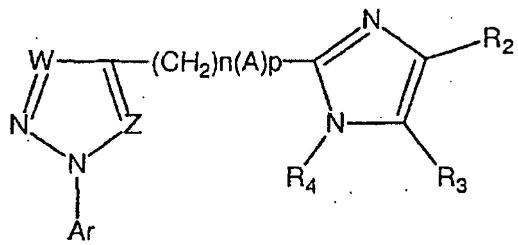

(I)

et les sels de celui-ci, dans laquelle

Ar est 2,6-dichloro-4-trifluorométhylphényle ou 2-nitro-4-trifluorométhylphényle ;
A est $S(O)_m$, -CH=CH-, O ou NH ;
W est N et Z est $CR^5$ ; ou W est $CR^1$ et Z est N ou $CR^5$ ;
$R^1$ est hydrogène, alkyle éventuellement substitué, halogène ou $R^{20}S(O)_q$ ;
$R^2$ et $R^3$ sont hydrogène, alkyle, alcényle ou alcynyle, dont chacun est éventuellement substitué, aryle, cyano, halogène, nitro, $YR^{20}$ $S(O)_2NR^8R^9$, CHO, $NR^8R^9$ ou $CYNR^8R^9$ ;
$R^4$ est hydrogène, alkyle éventuellement substitué, alcényle éventuellement substitué, acyle ou alcoxycarbonyle éventuellement substitué ;
$R^5$ est hydrogène, alkyle, amino éventuellement substitué ou halogène ;
$R^8$ et $R^9$ sont identiques ou différents et sont hydrogène, alkyle éventuellement substitué, acyle ou aryle ;
$R^{20}$ est alkyle éventuellement substitué ;
Y est O ou S ;
m est 0 , 1 ou 2 ;
p est 0 ou 1 ;
n est 0, 1 ou 2 ; et
q est 0 , 1 ou 2 ,

et dans laquelle a) tout groupement alkyle, alcoxy et alkylthio est constitué de 1 à 4 atomes de carbone ; b) tout groupement alcényle ou alcynyle est constitué de 2 à 5 atomes de carbone ; c) tout groupement alkyle substitué,

alcoxy, alkylthio, alcényle ou alcynyle est substitué par un ou plusieurs groupements identiques ou différents choisis parmi halogène, $YR^{20}$, dihalogénocyclopropyle, cyano, nitro, amino éventuellement substitué, acyloxy et aryle ; d) tout groupement aryle est phényle, éventuellement substitué par halogène, alkyle, halogénoalkyle, alcoxy, halogénoalcoxy, alkylthio, halogénoalkylthio, halogénoalkylsulfonyle, cyano ou nitro ; e) tout groupement acyle est alcanoyle constitué de 1 à 4 atomes de carbone, ou alkylsulfonyle ou halogénoalkylsulfonyle ; et f) tout groupement amino éventuellement substitué est de formule $NR^8R^9$, à condition que lorsque W est $CR^1$ et Z est $CR^5$ et n et p sont tous deux 0, $R^4$ ne soit pas alkyle.

2. Formulation selon la revendication 1, **caractérisée en ce que** le composé de formule (I) est le 5-chloro-1-(2,6-dichloro-4-trifluorométhylphényl)4-(4,5-dicyano-1H-imidazol-2-yl-3-méthyl-1H-pyrazole (composé 22c).

3. Formulation selon la revendication 1 ou 2, **caractérisée en ce que** les spinosynes sont un mélange de deux composés spinosyne quelconques ou plus.

4. Formulation selon la revendication 3, **caractérisée en ce que** le mélange est le spinosad.

5. Formulation selon l'une quelconque des revendications 1 à 4, **caractérisée en ce que** le composé de formule (I) et une ou plusieurs spinosynes sont présents dans un rapport de 1:10 à 10:1.

6. Utilisation d'une ou plusieurs spinosynes et d'au moins un composé de formule (I) en tant que principes actifs dans la fabrication d'un médicament destiné à lutter contre une infestation par des ectoparasites en administrant les principes actifs en combinaison, soit simultanément soit séquentiellement.

7. Utilisation selon la revendication 6, **caractérisée en ce que** les principes actifs sont le 5-chloro-1-(2,6-dichloro-4-trifluorométhylphényl)-4-(4,5-dicyano-1H-imidazol-2-yl-3-méthyl-1-H-pyrazole et le spinosad.

8. Utilisation selon la revendication 6 ou 7, **caractérisée en ce que** le médicament est destiné à lutter contre une infestation par des ectoparasites en administrant les principes actifs simultanément.

9. Utilisation selon la revendication 8, **caractérisée en ce que** le médicament comprend les principes actifs dans une seule préparation.

10. Utilisation selon la revendication 6, **caractérisée en ce que** les ectoparasites sont les tiques.

11. Utilisation selon la revendication 6, **caractérisée en ce que** les ectoparasites sont les puces.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5571901 A
- WO 0111963 A
- EP 412849 A
- US 5362634 A


### Non-patent literature cited in the description


- *DowElanco trade magazine Down to earth,* 1997, vol. 52 (1